Europäisches Patentamt

European Patent Office

Office européen des brevets

(19)

(11) **EP 0 755 660 A2**

(12) **EUROPEAN PATENT APPLICATION**

(43) Date of publication:
 **29.01.1997 Bulletin 1997/05**

(51) Int Cl.$^6$: **A61B 19/00**

(21) Application number: **96305492.9**

(22) Date of filing: **26.07.1996**

(84) Designated Contracting States:
 **AT BE CH DE DK ES FR GB GR IE IT LI NL PT SE**

(30) Priority: **28.07.1995 GB 9515509**

(71) Applicant: **Armstrong Healthcare Limited
 High Wycombe, Bucks HP13 6EQ (GB)**

(72) Inventor: **Finlay, P.A.
 Beaconsfield, Bucks, HP9 1AE (GB)**

(74) Representative: **Frankland, Nigel Howard
 FORRESTER & BOEHMERT
 Franz-Joseph-Strasse 38
 80801 München (DE)**

(54) **An alignment apparatus**

(57) An alignment apparatus, the apparatus comprising positioning means supporting a linear cannula or guide, and adapted to position the cannula or guide, the cannula or guide, or an optional linear, a probe within the cannula, having an end and having at least one point marked thereon so as to be visible in an X-ray image thereof, an X-ray apparatus adapted to take X-ray images in two orthogonal planes, a display adapted to display said X-ray images, means to identify, on the displayed images, a line with which the probe is to be aligned, the display being associated with a computer, means being provided to store, in the memory of the computer, data concerning specific selected points on the display, which points comprise at least points present on the image of the probe present on the display and points present on a line on the display with which the probe is to be aligned, the computer being adapted to determine the movement to be effected by the positioning means to align the cannula with the desired line.

FIG 1

## Description

THE PRESENT INVENTION relates to an alignment apparatus, for use when seeking to align a guide or cannula with a predetermined direction. One embodiment of the invention comprises a robotically controlled apparatus intended to align a guide or cannula with a predetermined direction.

Robotically controlled apparatus is now finding many applications and, in particular, is finding applications within the operating theatre. It is often desired to align a cannula with a predetermined direction and to insert a probe or operating implement into a patient by a predetermined distance through the cannula. For example, in connection with hip operations, it is often desirable to be able to drill into the top part of the femur along a predetermined direction. This is but one application of a robotically controlled apparatus in the operating theatre.

The present invention seeks to provide a robotically controlled apparatus that can align a cannula with a predetermined direction.

According to this invention there is provided an alignment apparatus, the apparatus comprising positioning means supporting a linear cannula or guide, and adapted to position the cannula or guide, the cannula or guide, or an optional linear, a probe within the cannula, having an end and having at least one point marked thereon so as to be visible in an X-ray image thereof, an X-ray apparatus adapted to take X-ray images in two orthogonal planes, a display adapted to display said X-ray images, means to identify, on the displayed images, a line with which the probe is to be aligned, the display being associated with a computer, means being provided to store, in the memory of the computer, data concerning specific selected points on the display, which points comprise at least points present on the image of the probe present on the display and points present on a line on the display with which the probe is to be aligned, the computer being adapted to determine the movement to be effected by the positioning means to align the cannula with the desired line.

Preferably control means are provided to enable an operator to control the position of a marker on said display, so that the marker can be moved to points in the image, the positions of which are to be stored in the memory of the computer.

Conveniently the control means can be used to create, and display, the line with which the probe is to be aligned.

In one embodiment of the positioning means comprise a robot, said computer controlling the robot.

In order that the invention may be more readily understood, and so that further features thereof may be appreciated, the invention will now be described, by way of example, with reference to the accompanying drawings in which

FIGURE 1 is a diagrammatic view of an X-ray apparatus,

FIGURE 2 is a diagrammatic view of a robotically controlled apparatus incorporating a cannula and the X-ray apparatus of Figure 1,

FIGURE 3 is a representation of one display that may be shown on the screen of the display device of Figure 2, and

FIGURE 4 is a representation of another display that may be shown on the display of the display device of Figure 2.

FIGURE 5 is a diagrammatic view corresponding to Figure 3 provided for purposes of explanation, and

FIGURE 6 is a diagrammatic view corresponding to Figure 4 provided for purposes of explanation.

The present invention will be described, with reference to the drawings, in the context of a robotically controlled apparatus.

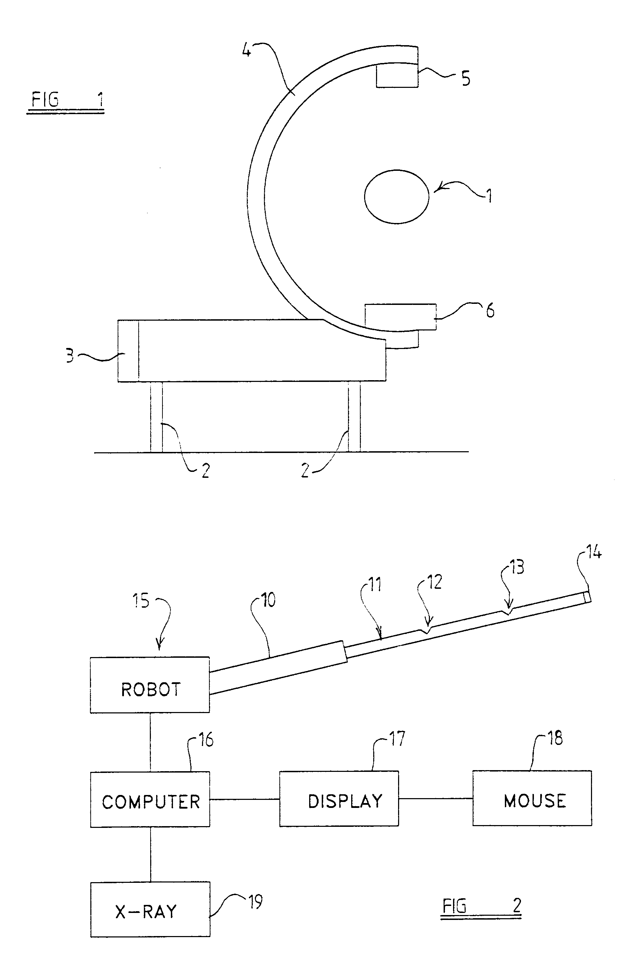

Referring initially to Figure 1, a component of a robotically controlled apparatus is an X-ray device capable of taking X-ray images of a patient 1 in two orthogonal directions. In the apparatus illustrated in Figure 1, the apparatus consists of a support 2 which supports a housing 3 containing a drive mechanism. The drive mechanism is adapted to drive a substantially "C"-shaped support frame 4 between two alternate positions. In the first position of the "C"-frame 4 an X-ray emitter 5 carried on the support frame is located substantially above the patient 1 and an X-ray receiving sensor 6 is located beneath the patient. The X-ray sensor 6 is provided with a cross-wire which lies in the plane of the "C"-frame 4.

The apparatus of Figure 1 is such that the "C"-frame 4 may be moved to an alternate position in which the X-ray emitter 5 and the X-ray sensor 6 are located on either side of the patient, lying in a horizontal plane. Thus it is to be understood that in one position of the "C"-frame, a "vertical" X-ray view (sometimes called an Anterior-Posterior, or AP view) of the patient may be obtained whereas when the "C"-frame is in the alternate position, a "horizontal" X-ray view (sometimes called the Lateral or LAT view) of the patient may be obtained. Alternative X-ray arrangements may be provided which give the same effect.

Figure 2 is a diagrammatic representation of a positioning means, which comprises a robot which supports a cannula or guide, and an associated probe, and which can position the cannula and probe. Referring to Figure 2, a cannula 10 is illustrated which is a linear element which contains a telescopically movable linear probe 11. In this embodiment, the probe 11 is provided with two notches 12,13 in its outer surface, which are provided so as to define points on the probe which are visible in an X-ray image of the probe for a purpose that will become clear later. It is preferred that the distance between the notch 13 and the end 14 of the probe is at least substantially equal to the distance between the notch 12 and the notch 13. Alternatively, the probe may be omitted and the markings may be provided on the cannula.

The cannula 10 and the degree of telescopic extension of the probe 11 relative to the cannula 10 are controlled by a robot 15. The precise nature of the robot is not critical to the present invention.

The robot is controlled by a computer 16 which has a display screen 17 and which is also associated with a mouse or other control arrangement 18. An X-ray apparatus 19, such as the X-ray apparatus illustrated in Figure 1 is connected to the computer, such that the image received by the X-ray sensor 6 can be displayed on the screen of the computer.

In use of the apparatus the robot is initially activated to bring the probe 11 into approximate alignment with the part of the patient 1 where an operating tool is to be introduced into the patient so that the probe is located between the X-ray emitter 5 and the X-ray sensor 6. X-ray images, both "vertical" and "horizontal", are taken of the part of the patient to be treated and the probe. The "C"-frame 4 is rotated by 90° after the first image is taken and before the second image is taken. The images are displayed sequentially or simultaneously upon the computer screen. The mouse 18 is used to control the position of a marker on the display screen and the marker is brought into alignment with various parts of the displayed image under the control of the operator of the system, and the mouse is activated to store, within the appropriate memory of the computer, information regarding the various parts of the image. The computer is then able to determine the position of the probe relative to the position that it is to adopt, and can move the probe appropriately.

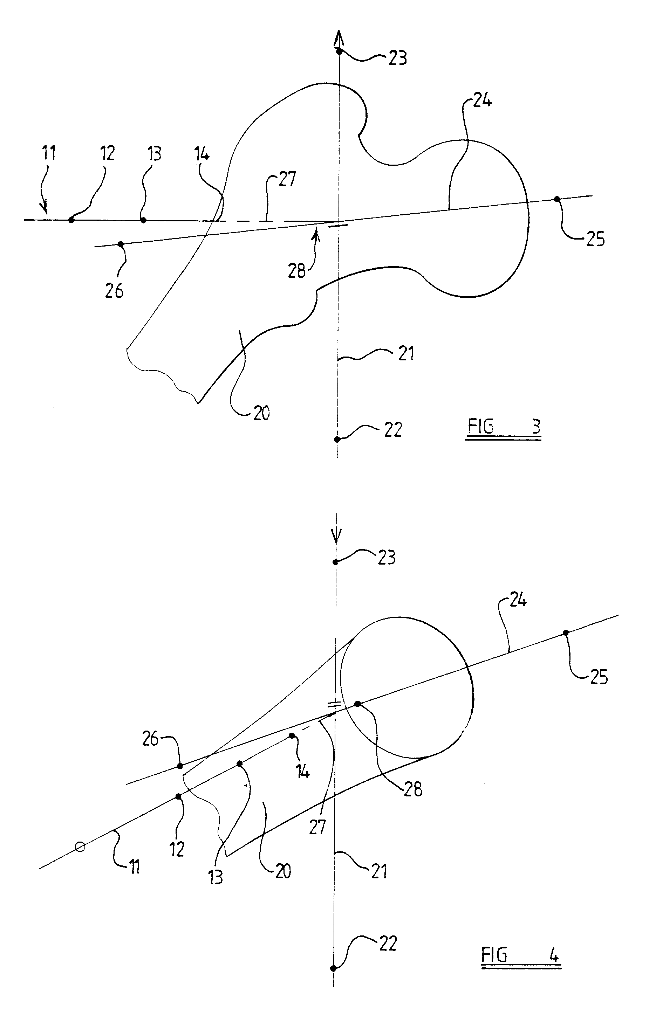

Referring, for example, to Figure 3, which is a "vertical" view (or "AP view") of part of a patient, the top part of the femur 20 of the patient is visible. The cross-wire 21 that is present on the sensor 6 can also be seen, and the probe 11 is also visible. The end 14 of the probe is clearly visible as are the notches 12 and 13.

Using the mouse to control an appropriate marker on the screen of the display 17, the operator of the device traces the line of the probe 11 indicating at least the positions on the screen of the notches 12,13 and the end of the probe 14. Also, the position of the wire 21 on the screen is indicated, possibly by indicating two spaced apart points 22,23 present on the wire. Also marked on the screen is a line 24, which is an indication of the line with which the probe is eventually to be aligned. This line may be selected by a surgeon to indicate, for example, the precise line on which a bore is to be drilled. This line may be called "the surgeon's line". Two specific separated points 25,26 may be particularly indicated. The computer 16 may be programmed to generate, upon the screen, a line 25 representing an extension of the probe 11. This extension may intersect the line 24 at a point 26. The point 26 may also be specifically indicated to the computer by use of the mouse, but the computer program may itself be capable of determining this particular point.

Figure 4 is a representation of the image on the screen of the display when the "horizontal" X-ray view (or "LATERAL view") is taken. Again the probe 11 can be seen, and again the end 14 of the probe is clearly visible together with the notches 12 and 13. The same procedure as adopted when the display of Figure 3 was on the screen is followed, the mouse and in indicator on the screen again being used to enable the computer to store, within its memory, information concerning the position of the probe 11. Again the position of the wire 21 on the screen is indicated, again by possibly identifying clearly the position of the points such as the points 22 and 23. Again, the line 24 with which the cannula is to be aligned (the "surgeon's line") may effectively be recorded within the memory of the computer, particularly by storing information concerning the position of the points 25 and 26. Again, the computer may generate, upon the screen, a line 27 representing an extension of the probe 11, with the line 27 intersecting the line 24 at the point 28, but the point 28 may again be indicated by use of the marker on the screen as controlled by the mouse.

The computer program is adapted to calculate the absolute position of the line 24 which, because it has been defined in two orthogonal planes (i.e. the plane of Figure 3 and the plane of Figure 4) has been defined absolutely. The computer then calculates the repositioning that is necessary for the cannula to align the cannula accurately with the line 24. The robot then executes the appropriate movement.

Once the cannula has been aligned with the line 24, the X-ray procedure may be repeated to ensure that the cannula is indeed accurately aligned with the intended line of operation. An appropriate operating tool may then be inserted into the patient through the cannula.

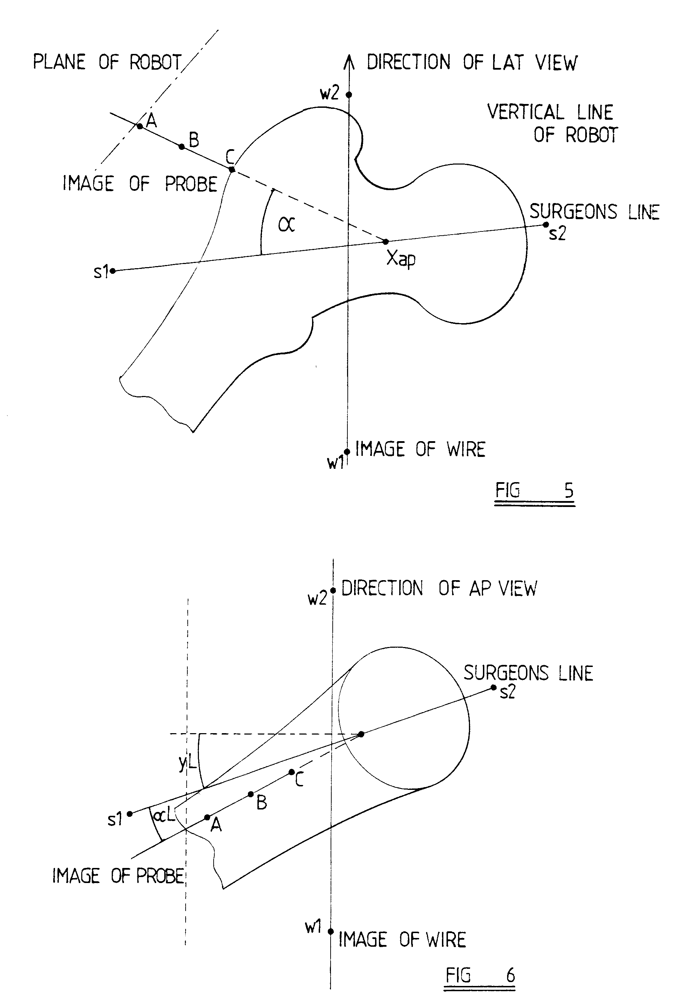

Whilst various procedures may be utilised to enable the robot to align the probe with the desired line or "surgeon's line", one procedure will be described and outlined below with reference to Figures 5 and 6 which correspond essentially with Figures 3 and 4, but which identify the relevant parts of the drawings in mathematical terms.

Figure 5 is the "AP view" and Figure 6 is the "LAT view".

The initial calculations from information read from the screen are the same for both AP and LAT views:

1) Let the screen co-ordinates of A, B and C be $(x_A, y_A)$, $(x_B, y_B)$ and $(x_C, y_C)$.

2) Let there be two points, S1 and S2, on the surgeons line, with screen co-ordinates $(x_{S1}, y_{S1})$ and $(x_{S2}, y_{S2})$.

3) Let there be two points, W1 and W2, on the image of the wire on the C-frame image intensifier, with screen co-ordinates $(x_{W1}, y_{W1})$ and $(x_{W2}, y_{W2})$.

4) Calculate $(x_A - x_C)$, $(y_A - y_C)$, $(x_A y_C - x_C y_A)$, $(x_{S1} - x_{S2})$, $(y_{S1} - y_{S2})$, $(x_{S1} y_{S2} - x_{S2} y_{S1})$, $[(y_{S1} - y_{S2})(x_A - x_C) - (y_A - y_C)(x_{S1} - x_{S2})]$.

5) Then, the screen co-ordinates of X, the point of intersection of the image of the probe (or its extension) with the surgeons line, are:

$$x_x = \frac{(x_{S1}y_{S2} - x_{S2}y_{S1})\,(x_A - x_C) - (x_A y_C - x_C y_A)\,(x_{S1} - x_{S2})}{(y_{S1} - y_{S2})\,(x_A - x_C) - (y_A - y_C)\,(x_{S1} - x_{S2})}$$

$$y_x = \frac{(x_{S1}y_{S2} - x_{S2}y_{S1})\,(x_A - x_C) - (x_A y_C - x_C y_A)\,(x_{S1} - x_{S2})}{(y_{S1} - y_{S2})\,(x_A - x_C) - (y_A - y_C)\,(x_{S1} - x_{S2})}$$

6) Calculate

$$\{ABXC\} = \frac{(x_B - x_A)\,(x_X - x_C)}{(x_A - x_C)\,(x_X - x_B)}$$

and then

$$lx = \frac{l_B}{[\,1 - 2\,\{ABXC\}\,]}$$

where $l_B$ is the physical distance of B from C, the end of the probe. It is assumed that this is also the physical distance of B from A.
Then, the distance along the probe from $(x_l, y_{lc}, d_l)$ at the robot to X is $(l - l_L - l_B + l_X)$.

7) Obtain the physical co-ordinates $(\underline{x}_x, \underline{y}_x, \underline{z}_x)$. The underscores are to distinguish between physical and image co-ordinates.

8) Calculate the angle, $\alpha$, between the surgeon's line and the image of the probe:

$$\alpha = A \tan 2((y_C - y_A), (x_C - x_A)) - A \tan 2((y_{S2} - y_{S1}), (x_{S2} - x_{S1}))$$

9) Calculate the angle, $\gamma$, between the surgeon's line and the perpendicluar to the image of the wire:

$$\gamma = A \tan 2((y_{S2} - y_{S1}),(x_{S2} - x_{S1})) - A \tan 2((y_{W1} - y_{W2}),(x_{W2} - x_{W1})) + \frac{\pi}{2}$$

N.B. There is an inverse tan common between $\alpha$ and $\gamma$.

**This is the end of the section common to the AP and LAT views**

From here, suffices will be used to distiguish results from the two views, viz:

$$(x_{XAP}, y_{XAP}, z_{XAP}), \alpha_{AP}, \gamma_{AP}$$

$$(x_{XL}, y_{XL}, z_{XL}), \alpha_L, \gamma_L$$

The underscores can now be dropped.

**For the AP view**

10) Calculate $\beta_{AP} = A \tan 2((x_2 - x_1),(d_1 + d_2)) + \tan(\beta_{AP} - \alpha_{AP})$.

11) Then,

$$new\_x1 = x_{XAP} + (z_{XAP} - d_1)\tan(\beta_{AP} - \alpha_{AP})$$

and

$$new\_x2 = x_{XAP} + (z_{XAP} + d_2)\tan(\beta_{AP} - \alpha_{AP})$$

These are the new robot x-co-ordinates required to bring the probe into the vertical plane indicated by the surgeon's line,

**For the LAT view**

12) Calculate $\tan \gamma_{LP} = \tan \gamma_L \bullet \cos \gamma_{AP}$.

13) Then,

$$new\_y1C = y_{XL} - (\tan\gamma_{LP})\sqrt{(new\_x1 - x_{XL})^2 + (z_{xl} - d_1)^2}$$

and

$$new\_y2C = y_{XL} - (\tan\gamma_{LP})\sqrt{(new\_x2 - x_{XL})^2 + (z_{XL} - d_2)^2}$$

14) Calculate,

$$X_2^2 = (new\_x2 - new\_x1)^2 + (d_1 + d_2)^2$$

and

$$\sec\theta = \sqrt{1 + \frac{(new\_y2C - new\_y1C)^2}{X_2^2}}$$

15) Then,

$$new\_y1 = new\_y1C - R \cdot \sec\theta$$

and

$$new\_y2 = new\_y2C - (R - r) \cdot \sec\theta$$

These are the new robot y-co-ordinates required to bring the probe to the indicated orientation in the vertical plane referenced to in (11) above.

Whilst, in the described embodiment of the invention, the mouse is used to control the position of a marker to identify, on the displayed images, the position of the probe and the position of the cross-wire, it is envisaged that in alternative embodiments of the invention the computer may be provided with an image processing and identifying capability. The computer may thus be able to process the image and identify both the probe (or the cannular) and also the cross-wire, without any human intervention. It will, however, still be necessary for the "surgeon's line" to be marked on the displayed images under human control.

Whilst, in the described embodiment of the invention, the positioning means which initially support the cannular or guide, and which can position the cannular or guide, comprises a robot, it is to be appreciated that an alignment apparatus in accordance with the invention may be utilised in conjunction with alternative forms of positioning means, such as a positioning means which is manually controlled. In such a situation, it is envisaged that the computer would determine the movement to be effected by the positioning means to align the cannular with the desired line, and would provide, on a display, an appropriate instruction. For example, the positioning means supporting the linear cannular many be provided with adjusting wheels, rotation of which controls the orientation of the linear cannular in three dimensional space. The computer, in such a situation may provide specific instructions as to which adjusting wheels are to be actuated, and the degree of rotation to be imparted to each adjusting wheel.

## Claims

1. An alignment apparatus, the apparatus comprising positioning means supporting a linear cannula or guide, and adapted to position the cannula or guide, the cannula or guide, or an optional linear, a probe within the cannula, having an end and having at least one point marked thereon so as to be visible in an X-ray image thereof, an X-ray apparatus adapted to take X-ray images in two orthogonal planes, a display adapted to display said X-ray images, means to identify, on the displayed images, a line with which the probe is to be aligned, the display being associated with a computer, means being provided to store, in the memory of the computer, data concerning specific selected points on the display, which points comprise at least points present on the image of the probe present on the display and points present on a line on the display with which the probe is to be aligned, the computer being adapted to determine the movement to be effected by the positioning means to align the cannula with the desired line.

2. An apparatus according to Claim 1 wherein control means are provided to enable an operator to control the position of a marker on said display, so that the marker can be moved to points in the image, the positions of which are to be stored in the memory of the computer.

3. An apparatus according to Claim 2 wherein the control means can be used to create, and display, the line with which the probe is to be aligned.

4. An alignment apparatus according to any one of the preceding Claims wherein the positioning means comprise a robot, and said computer controls the robot.

FIG 1

FIG 2

ROBOT

COMPUTER — DISPLAY — MOUSE

X-RAY

FIG 3

FIG 4

PLANE OF ROBOT

DIRECTION OF LAT VIEW

w2

VERTICAL LINE
OF ROBOT

A

B

C

IMAGE OF PROBE

SURGEONS LINE
s2

α

s1

Xap

IMAGE OF WIRE
w1

FIG 5

w2 DIRECTION OF AP VIEW

SURGEONS LINE
s2

yL

C

B

s1

αL

A

IMAGE OF PROBE

w1 IMAGE OF WIRE

FIG 6